# EUROPEAN PATENT APPLICATION

(11) **EP 1 291 017 A2**
(43) Date of publication of application: **12.03.2003**
(21) Application number: 02019026.0
(22) Date of filing: 27.08.2002
(51) Int. Cl.: A61K 31/366, A61K 31/22, A61K 31/4418, A61K 31/40, A61K 31/404, A61K 31/505, A61P 19/00, A61P 19/08, A61P 19/10, A61K 31/47

(54) **Use of statins to inhibit formation of osteoclasts**

(30) Priority: 10.09.2001 US 318450 P
(71) Applicant: WARNER-LAMBERT COMPANY, Morris Plains, New Jersey 07950 (US)
(72) Inventor: Baragi, Vijaykumar M., Michigan 48105 (US); Devalaraja, Radhika, Michigan 48103 (US); Peters, Brandon R., Michigan 48026 (US); Renkiewicz, Richard Raymond, Novi, Michigan 48375 (US)
(74) Representative: Tesch, Rudolf, Dr.

(57) **Abstract**

A method for inhibiting the formation of osteoclasts comprising administering a therapeutically effective amount of a statin to a mammal in need thereof as well as pharmaceutical compositions, kits for containing such compositions comprising a statin or a method of treating or preventing a disease state selected from the group consisting of: osteoporosis, Paget's disease, osteolysis, hypercalcemia of malignancy, osteogenesis imperfecta, osteoarthritis, alveolar bone loss, side effects of immunosuppressive therapy, and side effects of chronic glucocorticoid use by inhibiting the formation of osteoclasts comprising administering a therapeutically effective amount of a statin to a mammal in need thereof.

## Description

### FIELD OF THE INVENTION

The present invention relates to the use of statins to inhibit the formation of osteoclasts, in particular, the use of atorvastatin to inhibit the formation of osteoclasts as well as pharmaceutical compositions, kits for containing such compositions comprising a statin and a method of treating or preventing a disease, such as, for example, osteoporosis, Paget's disease, osteolysis, hypercalcemia of malignancy, osteogenesis imperfecta, osteoarthritis, alveolar bone loss, and side effects of immunosuppressive therapy and chronic glucocorticoid use by inhibiting formation of osteoclasts.

### BACKGROUND OF THE INVENTION

Maintenance of bone density is a dynamic process wherein osteoclasts resorb and osteoblasts resynthesize the bone, Parfitt AM, Riggs BL, Melton LJ, Osteoporosis: etiology, diagnosis and management, New York: Raven Press, 1988;13:501. An imbalance between resorption and formation leads to bone loss, in conditions such as osteoporosis and Paget's disease. Other conditions and diseases affected by this imbalance include osteolysis, hypercalcemia of malignancy, osteogenesis imperfecta, osteoarthritis, alveolar bone loss or side effects of immunosuppressive therapy and chronic glucocorticoid use. The bone loss is partly a consequence of enhanced osteoclastogenesis―a series of events that includes differentiation, migration, fusion, and survival of osteoclasts. Osteoclasts, the multinucleated giant cells, develop from hematopoietic cells of macrophage lineage as a result of increased production of osteoclastogenic cytokines by osteoblasts/stromal cells, Suda T, Takahashi N, Martin TJ, Modulation of osteoclast differentiation, *Endocr. Rev*., 1992;13:66-80; Chambers TJ, Revell PA, Fuller K, Athanasou NA, Resorption of bone by isolated rabbit osteoclasts, *J. Cell Sci.*, 1984;66:383-399; Takahashi N, Udagawa N, Suda T, A new member of TNF ligand family, ODF/OPGL/TRANCE/RANKL, regulates osteoclast differentiation and function, *Biochem. Biophys. Res. Commun*., 1999;256:449-455; and Suda T, Takahashi N, Udagawa N, Jimi E, Gillespie MT, Martin TJ, Modulation of osteoclast differentiation and function by the new members of the TNF receptor and ligand families. *Endocr. Rev*., 1999;20:345-357. Of the various osteoclastogenic cytokines, macrophage colony stimulating factor (M-CSF) and receptor activator of NF-kB ligand (RANKL) are considered to be essential for osteoclast development, Jimi E, et al., Osteoclast function is activated by osteoblastic cells through a mechanism involving cell-to-cell contact, *Endocrinology,* 1996;137:2187-2190; and Lacey D, et al., Osteoprotegerin ligand murine osteoclast survival *in vitro* and *in vivo*, *Am. J. Pathol*., 2000;157:435-448.

Several different therapeutic agents have been used to treat bone loss associated with osteoporosis; however, little is known about their mechanism of action. Recent studies indicate that perturbation of the cholesterol biosynthetic pathway has a direct effect on osteoclasts. Nitrogen-containing bisphosphonates, for example, are reported to inhibit formation and promote apoptosis of osteoclasts by preventing protein prenylation, a rate-limiting step in the cholesterol biosynthesis pathway, Cummings S, Bauer DC, Do statins prevent both cardiovascular disease and fracture?, *J*. *Am. Med. Assoc*., 2000;283:3255-3257; Wang PS, Solomon DH, Mogun H, Avorn J, HMG-CoA reductase inhibitors and the risk of hip fractures in elderly patients, *J. Am. Med. Assoc*., 2000;283:3211-3216; Meier CR, Schlienger RG, Kraenzlin ME, Schlegel B, Hershel J, HMG-CoA reductase inhibitors and the risk of fractures, *J. Am. Med. Assoc*., 2000;283:3205-3210; Russell RG, Rogers MJ, Frith JC, et al., The pharmacology of bisphosphanates and new insights into their mechanisms of action, *J. Bone Miner Res.,* 1999;14:53-65; Reszka AA, Halasy-Nagy JM, Masarachia PJ, Rodan GA, Bisphosphanates act directly on the osteoclast to induce caspase cleavage of mstl kinase during apoptosis: a link between inhibition of the mevalonate pathway and regulation of apoptosis-promoting kinase. *J*. *Biol. Chem*., 1999;274:34967-34973. Inhibition of HMG-CoA reductase, an early step in the cholesterol biosynthesis, by statins would be expected to have similar effects on osteoclasts. On the contrary, Mundy G, et al., Stimulation of bone formation *in vitro* and in rodents by statins, *Science,* 1999;286:1946-49 have shown that administration of statins *in vivo* increases bone morphogenic protein-2 (BMP-2) levels, which in turn results in increased bone formation. Similarly, simvastatin and lovastatin were reported to stimulate a greater dose-dependent increase in total bone area compared to established growth factors such as BMP-2 or basal fibroblast growth factor (b-FGF) in both normal and ovariectomized (OVX) rats (see US Patent No. *6,022,887*).

Thus, while bisphosphonates have been shown to be antiresorptive, available data seem to suggest that statins act as anabolic agents. There is little, if any, data to suggest an antiresorptive role for statins. Mundy G, et al., supra., alluded to the possibility of simvastatin having antiresorptive properties but demonstrated only a marginal reduction in osteoclast number (25%) in rodents. International Published Patent Application WO 99/45923 disclosed that lovastatin at 10 µM concentration inhibits tartrate-resistant acid phosphatase (TRAP) activity significantly in osteoblast-osteoclast cocultures.

However, none of the above disclosures have demonstrated a therapeutic effect of atorvastatin or, for that matter, a statin on the regulation of osteoclastogenesis and the conditions and diseases associated therewith.

Unlike the above mentioned studies, we have employed a protocol that is mechanistically suitable for examining the effects of compounds directly on osteoclastogenesis, independent of the osteoblast/stromal cell pathway. In particular, we have found that atorvastatin can be used to inhibit formation of osteoclasts *in vitro* and *in vivo.*

Atorvastatin calcium, disclosed in United States Patent Number *5,273,995,* which is incorporated herein by reference, is currently sold as Lipitor®, and has the chemical name [R-(R*,R*)]-2-(4-fluorophenyl)-β,δ-dihydroxy-5-(1-methylethyl)-3-phenyl-4-[(phenylamino)carbonyl]-1H-pyrrole-1-heptanoic acid calcium salt (2:1) trihydrate and the formula

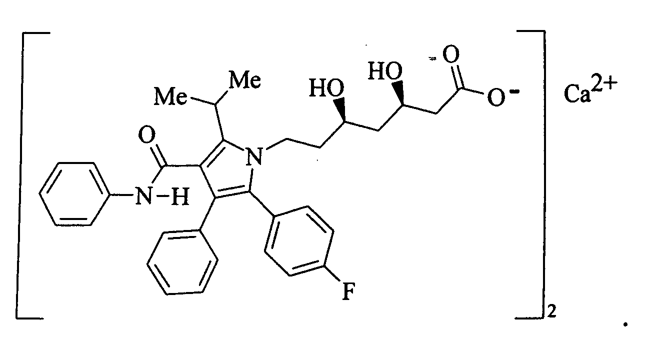

Atorvastatin calcium is a selective, competitive inhibitor of HMG-CoA reductase. As such, atorvastatin calcium is a potent lipid lowering compound and is thus useful as a hypolipidemic and/or hypocholesterolemic agent.

United States Patent Number *4,681,893*, which is incorporated herein by reference, discloses certain *trans*-6-[2-(3- or 4-carboxamido-substituted-pyrrol-1-yl)alkyl]-4-hydroxy-pyran-2-ones including trans (±)-5-(4-fluorophenyl)-2-(1-methylethyl)-N, 4-diphenyl-1-[(2-tetrahydro-4-hydroxy-6-oxo-2H-pyran-2-yl)ethyl]-1H-pyrrole-3-carboxamide.

United States Patent Number *5,273,995*, which is herein incorporated by reference, discloses the enantiomer having the R form of the ring-opened acid of *trans*-5-(4-fluorophenyl)-2-(1-methylethyl)-N, 4-diphenyl-1-[(2-tetrahydro-4-hydroxy-6-oxo-2H-pyran-2-yl)ethyl]-1H-pyrrole-3-carboxamide, i.e., [R-(R*,R*)]-2-(4-fluorophenyl)-β,δ-dihydroxy-5-(1-methylethyl)-3-phenyl-4-[(phenylamino)-carbonyl]-1H-pyrrole-1-heptanoic acid which is atorvastatin.

United States Patent Numbers *5,003,080*; *5,097,045*; *5,103,024*; *5,124,482*; *5,149,837*; *5,155,251*; *5,216,174*; *5,245,047*; *5,248,793*; *5,280,126*; *5,397,792*; *5,342,952*; *5,298,627*; *5,446,054*; *5,470,981*; *5,489,690*; *5,489,691*; *5,510,488*; *5,998,633*; and *6,087,511*, which are herein incorporated by reference, disclose various processes and key intermediates for preparing amorphous atorvastatin. Amorphous atorvastatin has unsuitable filtration and drying characteristics for large-scale production and must be protected from heat, light, oxygen, and moisture.

Crystalline forms of atorvastatin calcium are disclosed in United States Patent Numbers *5,969,156* and *6,121,461*, which are herein incorporated by reference.

International Published Patent Application WO 01/36384 purports to disclose a polymorphic form of atorvastatin calcium.

Stable oral formulations of atorvastatin calcium are disclosed in United States Patent Numbers *5,686,104* and *6,126,971*.

Surprisingly and unexpectedly, we have found that atorvastatin at relatively low concentrations (0.01 nM to 1 µM) significantly reduced the number of TRAP-positive, multinucleated cells in primary murine bone marrow cell cultures (IC₅₀ = 0.43 nM) as well as in RAW cells (IC₅₀ = 0.17 µM). Furthermore, *in vivo* studies using the ovariectomized rat model of bone loss indicated that daily oral administration of atorvastatin (2.5-10 mg/kg body weight) to rats over a 5-week period, significantly decreased the number of osteoclasts formed, as measured by histomorphometric analysis. Thus, we have found that atorvastatin has a direct effect on osteoclasts both *in vivo* and *in vitro* at physiological concentrations.

### SUMMARY OF THE INVENTION

Accordingly, the first aspect of the present invention is a method of inhibiting the formation of osteoclasts comprising administering a therapeutically effective amount of a statin to a mammal in need thereof.

A second aspect of the present invention is a pharmaceutical composition comprising a therapeutically effective amount of a statin and a carrier.

A third aspect of the present invention is a method of treating or preventing a disease state, such as, for example, osteoporosis, Paget's disease, osteolysis, hypercalcemia of malignancy, osteogenesis imperfecta, osteoarthritis, alveolar bone loss, side effects of immunosuppressive therapy, and side effects of chronic glucocorticoid use by inhibiting the formation of osteoclasts comprising administering a therapeutically effective amount of a statin to a mammal in need thereof.

A fourth aspect of the present invention is a therapeutic package or kit suitable for commercial sale, comprising a container and a pharmaceutical composition.

### BRIEF DESCRIPTION OF THE DRAWINGS

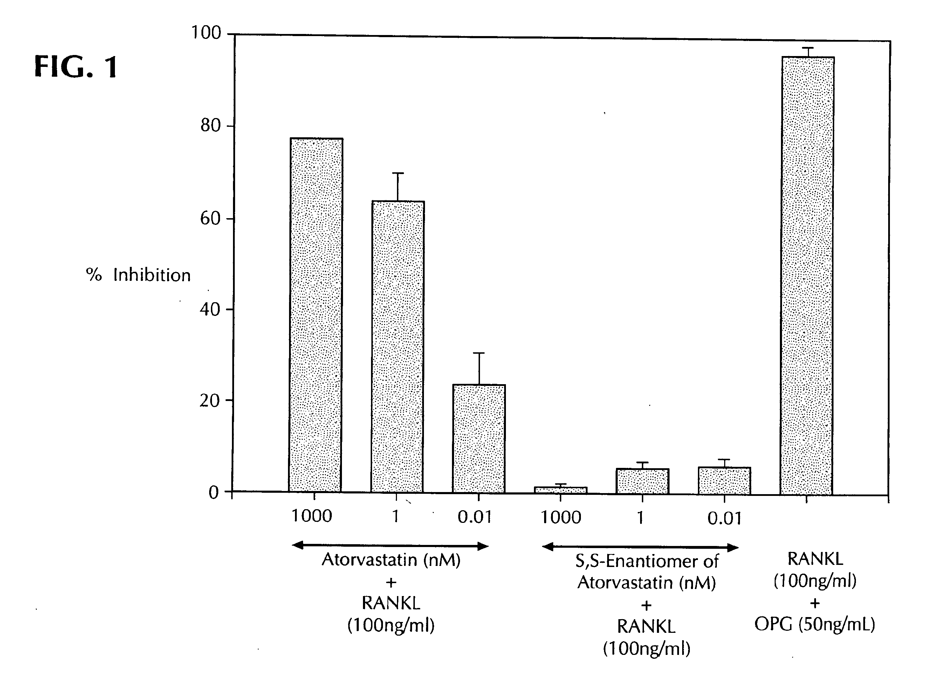

The present invention is further described by the following nonlimiting examples which refer to the accompanying Figures 1 to 4, short particulars of which are given below.

Figure 1. Effects of atorvastatin and the S,S-enantiomer of atorvastatin on RANKL-induced osteoclastogenesis.

Bone marrow cells were cultured with 100 ng/mL of M-CSF for 3 days. The cells were further cultured for 5 more days with either atorvastatin, its S,S-enantiomer or osteoprotegerin (OPG) in the presence of RANKL and M-CSF. Cells were then fixed and stained for TRAP. Cells with more than 3 nuclei were counted and the data expressed as % inhibition of TRAP-positive cells in cultures treated with RANKL alone. The data shown are mean ±SD (n = 4 wells/treatment). Similar results were obtained in 5 different experiments.

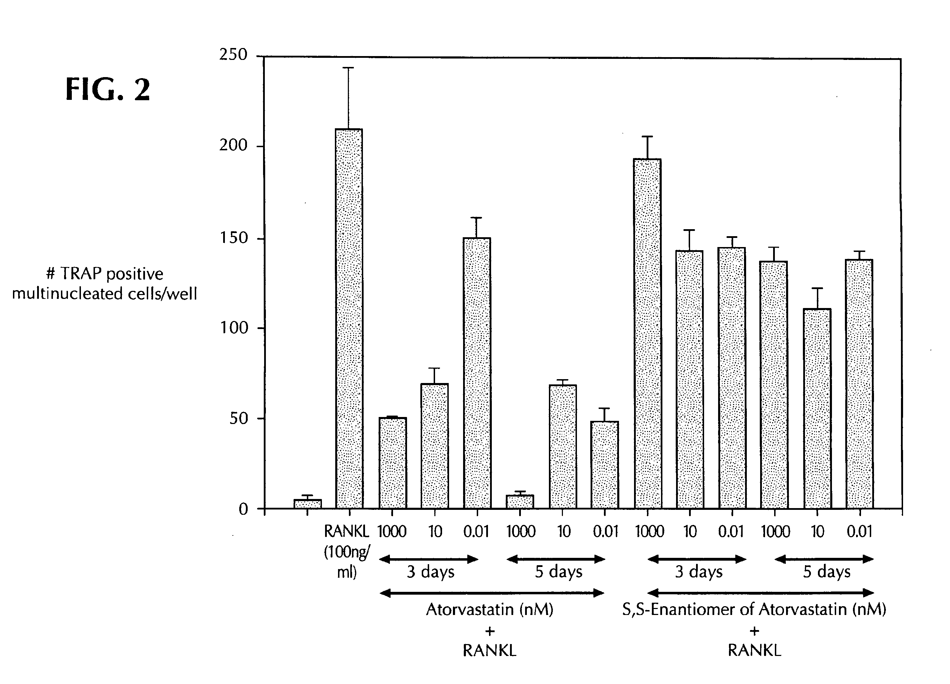

Figure 2. Time course effect of atorvastatin on osteoclastogenesis.

Bone marrow cells treated with M-CSF for 3 days were stimulated with RANKL as well as atorvastatin (0.01 nM, 10 nM, and 1 µM), its S,S-enantiomer or OPG. In some experiments atorvastatin was added 2 days post RANKL stimulation (5 days after isolation). On Day 8 postisolation, cells were fixed and stained for TRAP. TRAP-positive multinucleated cells were counted and represented. The data shown are mean of ±SD (n = 4 wells/treatment). Similar results were obtained in 3 different experiments.

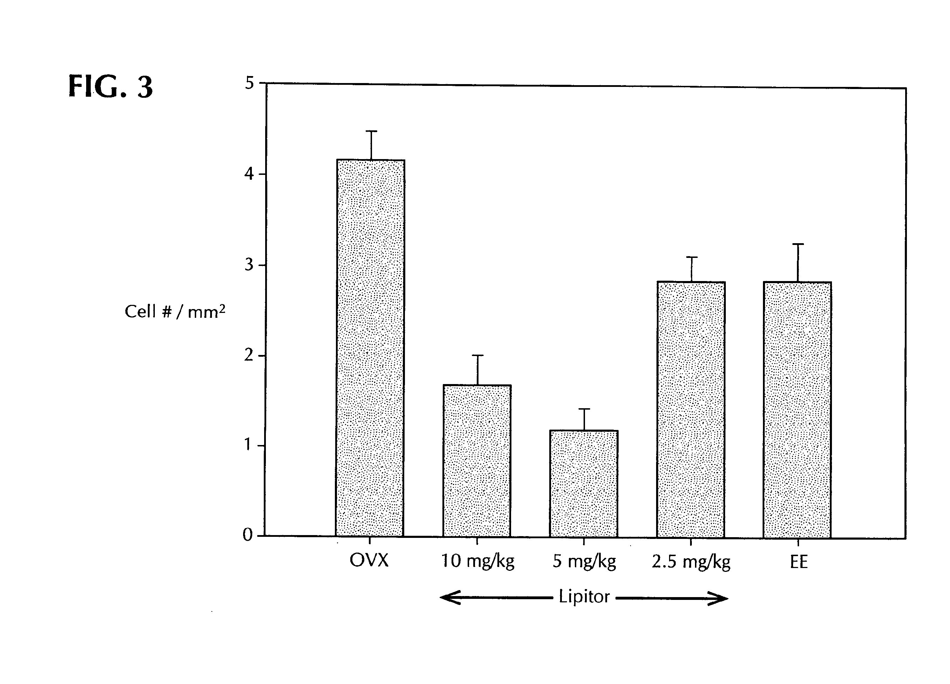

Figure 3. Atorvastatin treatment reduces osteoclast numbers in ovariectomized rats.

Rats were ovariectomized according to the standard protocol and were administered orally with various doses of atorvastatin (2.5, 5, and 10 mg/kg body weight) for 5 weeks. Tibiae were analyzed for osteoclast number by histomorphometry. Ethinyl estradiol (EE) (0.4 mg/kg), a known bone protective agent, was used as a positive control.

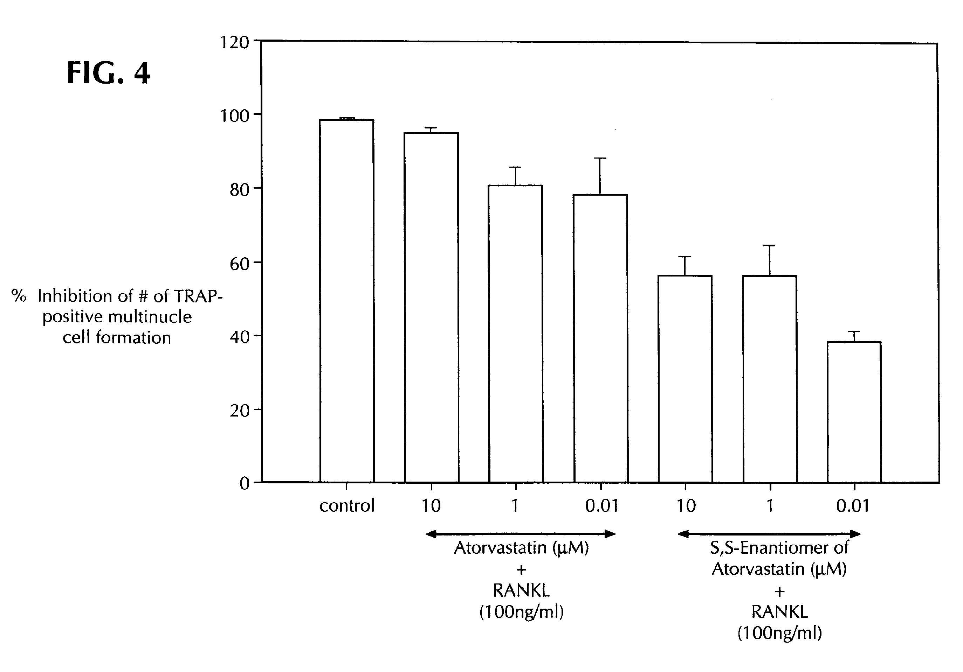

Figure 4. Effect of atorvastatin and its S,S-enantiomer on RANKL-induced osteoclastogenesis in RAW cells.

Raw cells (2000/well) were stimulated with RANKL (50 ng/mL) ± atorvastatin and S,S-enantiomer of atorvastatin for 5 days and TRAP staining was performed at the end of Day 5 and the data is expressed as % inhibition of number of TRAP-positive cells compared to control.

### DETAILED DESCRIPTION OF THE INVENTION

The present invention relates to a method of inhibiting the formation of osteoclasts comprising administering a therapeutically effective amount of a statin to a mammal in need thereof. The therapeutic regimen of the present invention is administered until the desired therapeutic effect is achieved.

The therapeutic agents useful in the present invention are statins.

Examples of statins that are useful herein include, but are not limited to, lovastatin (MEVACOR®; see US Patent No. *4,231,938*, which is incorporated by reference herein), simvastatin (ZOCOR®; see US Patent No. *4,444,784*, which is incorporated by reference herein); pravastatin (PRAVACHOL®; see US Patent No. *4,346,227,* which is incorporated by reference herein); fluvastatin (LESCOL®; see US Patent No. *5,354,772*, which is incorporated by reference herein); atorvastatin (LIPITOR®; see US Patent No. *5,273,995,* which is incorporated by reference herein); cerivastatin (also known as rivastatin; see US Patent No. *5,177,080*, which is incorporated by reference herein); mevastatin (compactin, see US Patent No. *3,983,140*, which is herein incorporated by reference); rosuvastatin (Crestor®, see US Patent No. 5,260,440, International Published Patent Application WO 00/42024, and Watanabe M. et al., *Biorg. Med. Chem*., 1997;5:437-447, which are incorporated by reference herein); and itavastatin (see US Patent No. 5,753,675, which is incorporated by reference herein). Preferably, the statin is selected from the group consisting of lovastatin, simvastatin, pravastatin, fluvastatin, atorvastatin, cerivastatin, mevastatin, rosuvastatin, itavastatin, and the pharmaceutically acceptable salts, esters, and lactones thereof, and mixtures thereof. Most preferably, the statin is atorvastatin calcium.

The term "S,S-enantiomer of atorvastatin" refers to (3S,5S)-7-[2-(4-fluorophenyl)-5-isopropyl-3-phenyl-4-phenylcarbamoyl-pyrrol-1-yl]-3,5-dihydroxy-heptanoic acid calcium salt (2:1), which is the inactive enantiomer of atorvastatin.

The term "esters" as used herein referring to a statin, is used in its standard meaning to denote the condensation product of a carboxylic acid and an alcohol. Ester derivatives of the described compounds can function as prodrugs which, when absorbed into the bloodstream of a warm-blooded animal, can cleave in such a manner as to release the drug form and permit the drug to afford improved therapeutic efficacy.

The term "lactones" as used herein in referring to a statin, is used in its standard meaning to denote a cyclic condensation product of a carboxylic acid and an alcohol, i.e., a cyclic ester.

The term "therapeutically effective amount," as used herein, means that amount of a statin that will elicit the desired biological or medical effect or response sought by a medical doctor, clinician, veterinarian, researcher, or other appropriate professional, when administered in accordance with the desired treatment regimen. A preferred therapeutically effective amount is an osteoclast inhibiting amount. The term "therapeutically effect amount" is also intended to encompass prophylactically effective amounts, i.e., amounts that are suitable for preventing a disease state or condition, if a prophylactic or prevention benefit is desired.

The term "until the desired therapeutic effect is achieved," as used herein, means that the statin is administered, according to the dosing schedule chosen, up to the time that the clinical or medical effect sought for the disease or condition being treated or prevented is observed by the clinician or researcher. For the methods of treatment of the present invention, the statin is continuously administered until the desired change in bone mass or structure is observed. In such instances, achieving an increase in bone mass or a replacement of abnormal bone structure with normal bone structure are the desired objectives. For methods of prevention of the present invention, the statin is continuously administered for as long as necessary to prevent the undesired condition or disease state. In such instances, maintenance of bone mass density is often the objective. Nonlimiting examples of treatment and prevention administration periods can range from about 2 weeks to the remaining lifespan of the mammal. For humans, administration periods can range from about 2 weeks to the remaining lifespan of the human, preferably from about 2 weeks to about 20 years, more preferably from about 1 month to about 20 years, more preferably from about 6 months to about 10 years, and most preferably from about 1 year to about 10 years.

The term "mammal" includes humans.

### Effect of Atorvastatin on Osteoclast Formation In Vitro

Bone marrow cells were cultured in the presence of 100 ng/mL of recombinant murine M-CSF for 3 days after isolation. The cells were then stimulated with murine RANKL (100 ng/mL) in the presence of various doses of atorvastatin (0.01 nM, 1 nM, and 1 µM), S,S-enantiomer of atorvastatin, or osteoprotegerin (OPG) and further cultured for 5 more days and stained for TRAP activity (see Figure 1). The number of TRAP-positive multinucleated cells formed by RANKL was completely inhibited by the addition of 50 ng/mL of OPG simultaneously to bone marrow derived osteoclasts.

### Effect of Atorvastatin on Osteoclastogenesis in RAW 264.7 Cells

RAW cells were plated at a density of 2000 cells/well in a 48-well plate and cultured overnight in alpha-MEM medium containing antibiotics. Cells were treated with either RANKL (50 ng/mL) ± atorvastatin or the S,S-enantiomer of atorvastatin (10, 1, and 0.01 µM) for 5 days and stained for TRAP enzyme. Purple stained cells with three or more nuclei were counted under the microscope and represented as % inhibition of osteoclastogenesis compared to control cells. Thus, in Figure 4, column 1 indicates that control cells barely had any TRAP cell formation as indicated by almost 100% inhibition. Columns 2, 3, and 4 indicate that atorvastatin even at 0.01 micromolar treatment had almost 80% inhibition of RANKL-induced multinucleated cell formation, whereas, its S,S-enantiomer (columns 4, 6, and 7) showed significantly less % inhibition.

### Time-Course of Inhibition of RANKL-Induced Osteoclastogenesis by Atorvastatin

Osteoclastogenesis involves a series of events which include differentiation, proliferation, activation, and fusion to form multinucleated cells that are capable of resorbing bone. To examine the stage at which atorvastatin inhibits osteoclastogenesis, atorvastatin was added at various times to mouse bone marrow cells in the presence of M-CSF and RANKL. Figure 2 indicates that addition of atorvastatin 2 days post RANKL stimulation (5 days after isolation) was able to block the number of TRAP-positive cells formed. On the other hand, no differences were observed when the S,S-enantiomer of atorvastatin was added to the cell cultures. Interestingly, the effects of atorvastatin when added on Day 5 were much more pronounced than when added on Day 3 postisolation. This could be because the atorvastatin added on Day 3 was perhaps inactivated to some extent than when added on Day 5, the time when the fusion and activation process occurs. These results suggest that atorvastatin, by acting directly on osteoclasts, could be modulating the final fusion/activation step crucial for multinucleated cell formation.

### Effect of Atorvastatin on Osteoclast Numbers in Ovariectomized Rats

Ovariectomy-induced bone loss in rats is an established model of osteoporosis, wherein estrogen insufficiency leads to increased osteoclastogenesis, possibly due to enhanced cytokine production. To test whether statins decrease bone resorption *in vivo,* ovariectomized rats were given orally various doses of atorvastatin (2.5, 5, or 10 mg/kg body weight) for 5 weeks. At the end of the study, the animals were sacrificed, and histomorphometric analysis was performed on tibia obtained from the animals. The data revealed the presence of significantly greater numbers of osteoclasts (Figure 3) in ovariectomized animals compared to the sham controls. Administration of atorvastatin at 5 or 10 mg/kg to ovariectomized rats significantly decreased the osteoclast numbers. Atorvastatin at both 5 and 10 mg/kg doses was more effective in decreasing the osteoclast formation than ethinyl estradiol, a positive control used in this study. Thus, these data clearly demonstrate that statins have a direct inhibitory effect on the formation of osteoclasts.

The compounds of the present invention can be prepared and administered in a wide variety of oral and parenteral dosage forms. Thus, the compounds of the present invention can be administered by injection, that is, intravenously, intramuscularly, intracutaneously, subcutaneously, intraduodenally, or intraperitoneally. Also, the compounds of the present invention can be administered by inhalation, for example, intranasally. Additionally, the compounds of the present invention can be administered transdermally. It will be obvious to those skilled in the art that the following dosage forms may comprise as the active component, either compounds or a corresponding pharmaceutically acceptable salt of a compound of the present invention.

For preparing pharmaceutical compositions from the compounds of the present invention, pharmaceutically acceptable carriers can be either solid or liquid. Solid form preparations include powders, tablets, pills, capsules, cachets, suppositories, and dispersible granules. A solid carrier can be one or more substances which may also act as diluents, flavoring agents, solubilizers, lubricants, suspending agents, binders, preservatives, tablet disintegrating agents, or an encapsulating material.

In powders, the carrier is a finely divided solid which is in a mixture with the finely divided active component.

In tablets, the active component is mixed with the carrier having the necessary binding properties in suitable proportions and compacted in the shape and size desired.

The powders and tablets preferably contain from 2% or 10% to about 70% of the active compound. Suitable carriers are magnesium carbonate, magnesium sterate, talc, sugar, lactose, pectin, dextrin, starch, gelatin, tragacanth, methylcellulose, sodium carboxymethylcellulose, a low melting wax, cocoa butter, and the like. The term "preparation" is intended to include the formulation of the active compound with encapsulating material as a carrier providing a capsule in which the active component, with or without other carriers, is surrounded by a carrier, which is thus in association with it. Similarly, cachets and lozenges are included. Tablets, powders, capsules, pills, cachets, and lozenges can be used as solid dosage forms suitable for oral administration.

For preparing suppositories, a low melting wax, such as a mixture of fatty acid glycerides or cocoa butter, is first melted, and the active component is dispersed homogeneously therein, as by stirring. The molten homogenous mixture is then poured into convenient sized molds, allowed to cool, and thereby to solidify.

Liquid form preparations include solutions, suspensions, retention enemas, and emulsions, for example, water or water propylene glycol solutions. For parenteral injection, liquid preparations can be formulated in solution in aqueous polyethylene glycol solution.

Aqueous solutions suitable for oral use can be prepared by dissolving the active component in water and adding suitable colorants, flavors, and stabilizing and thickening agents as desired.

Aqueous suspensions suitable for oral use can be made by dispersing the finely divided active component in water with viscous material, such as natural or synthetic gums, resins, methylcellulose, sodium carboxymethylcellulose, and other well-known agents.

Also included are solid form preparations which are intended to be converted, shortly before use, to liquid form preparations for oral administration. Such liquid forms include solutions, suspensions, and emulsions. These preparations may contain, in addition to the active component, colorants, flavors, stabilizers, buffers, artificial and natural sweeteners, dispersants, thickeners, solubilizing agents, and the like.

The pharmaceutical preparation is preferably in unit dosage form. In such form, the preparation is subdivided into unit doses containing appropriate quantities of the active component. The unit dosage form can be a packaged preparation, the package containing discrete quantities of preparation, such as packeted tablets, capsules, and powders in vials or ampoules. Also, the unit dosage form can be a capsule, tablet, cachet, or lozenge itself, or it can be the appropriate number of any of these in packaged form.

The quantity of active component in a unit dose preparation may be varied or adjusted from 0.5 mg to 100 mg, preferably 2.5 mg to 80 mg according to the particular application and the potency of the active component. The composition can, if desired, also contain other compatible therapeutic agents.

In therapeutic use as an agent to inhibit the formation of osteoclasts, and as an agent to treat or prevent a disease state selected from the group consisting of: osteoporosis, Paget's disease, osteolysis, hypercalcemia of malignancy, osteogenesis imperfecta, osteoarthritis, alveolar bone loss, side effects of immunosuppressive therapy, and side effects of chronic glucocorticoid use a statin and, in particular, atorvastatin utilized in the pharmaceutical method of this invention is administered at the initial dosage of about 2.5 mg to about 80 mg daily. A daily dose range of about 2.5 mg to about 20 mg is preferred. The dosages, however, may be varied depending upon the requirements of the patient, the severity of the condition being treated, and the compound being employed. Determination of the proper dosage for a particular situation is within the skill of the art. Generally, treatment is initiated with smaller dosages which are less than the optimum dose of the compound. Thereafter, the dosage is increased by small increments until the optimum effect under the circumstance is reached. For convenience, the total daily dosage may be divided and administered in portions during the day if desired.

Furthermore, the present invention relates to the treatment of diseases and conditions in a subject as described above that may be administered in a dosage form contained in a therapeutic package or kit. The kit includes the dosage form and a container. Typically, the kit includes directions for the administration of the dosage form. The container can be in any conventional shape or form as known in the art, for example, a paper box, a glass or plastic bottle, or a blister pack with individual dosage for pressing out of the back according to a therapeutic schedule.

The following nonlimiting examples illustrate the inventors' preferred methods for carrying out the present invention.

### EXAMPLE 1

### Bone Marrow Cell Cultures

Osteoclasts were obtained from bone marrow cells as described previously from 6 to 8 weeks old C57B1/6 mice, Kobayashi K, et al., Tumor Necrosis Factor α stimulates osteoclast differentiation by a mechanism independent of the ODF/RANKL-RANK interaction, *J. Exp. Med*., 2000;191:275-285. Briefly, after removing the femur aseptically, the adherent soft tissue was separated from the bone. Using a syringe and a needle, the marrow was flushed using alpha minimum essential medium (α-MEM). Osteoclasts were obtained by culturing bone marrow cells (0.15 × 10⁶ cells/well) in α-MEM containing 10% fetal bovine serum (FBS), 100 IU penicillin, 100 µg/mL of streptomycin and 100 ng/mL of recombinant murine M-CSF for 3 days in 48-well plates. The osteoclasts thus obtained were further cultured with RANKL ± atorvastatin, S,S-enantiomer of atorvastatin or osteoprotegerin (OPG), for 5 days. OPG is a soluble decoy receptor for RANKL that has previously been shown to be effective in blocking RANKL-induced osteoclastogenesis in several *in vivo* and *in vitro* models. In some experiments, the compounds were added on Day 5 postisolation. At the end of Day 5, cells were stained for TRAP activity using the Leukocyte Acid Phosphatase kit from Sigma Chemical company, Goldberg AF and Barka T, Acid phosphatase activity in human blood cells, *Nature,* 1962;189:297.

### EXAMPLE 2

### RAW 264.7 Cell Cultures

RAW cells were cultured in α-MEM containing 10% FBS, 100 IU penicillin, 100 µg/mL of streptomycin. Cells were plated at a density of 2000 cells/well in a 48-well plate. After overnight incubation, the cells were stimulated with RANKL (50 ng/mL) ± atorvastatin, S,S-enantiomer of atorvastatin or OPG for 5 days and stained for TRAP at the end of Day 5.

### EXAMPLE 3

### Tartrate-Resistant Acid Phosphatase (TRAP) Staining

After initial wash with 1 × PBS, the cells were fixed for 30 seconds using acetone based fixing solution containing acetone, citric acid and 37% formaldehyde. After washing twice with distilled water that was prewarmed to 37°C, the cells were incubated in acetate buffer containing naphthol AS-B1 and Fast Garnet GBC solution for 45 minutes at 37°C. TRAP-positive cells are typically brown in color when viewed under the microscope. The total number of TRAP-positive cells with 3 or more nuclei in each well were counted. TRAP activity is a phenotypic marker of mature osteoclasts.

### EXAMPLE 4

### Ovariectomy and Histomorphometric Analysis

The rats were ovariectomized according to the standard protocol. Briefly, after anesthesia the rats were cleaned with antiseptic solution. A small incision was made on the back of the animal, and the ovaries were located carefully underneath the fat tissue. After proper clamping, ovaries were removed with a scalpel blade. Also, the fallopian tube with the uterine horn was sealed by electrocautery. Later, the skin incision was closed using wound clips. After recovery from the surgery, animals were randomized into 6 groups of 8 animals each (sham operated, OVX, OVX+ atorvastatin 2.5, 5, 10 mg/kg body weight and OVX+ ethinyl estradiol) and dosed with various concentrations of atorvastatin and vehicle control every day for a period of 5 weeks.

Histomorphometric analysis was performed using decalcified sections of the proximal tibia for cancellous bone and undecalcified sections for cortical bone. Briefly, tibiae were decalcified using 10% ethylene diaminetetraacetic acid (EDTA), followed by rehydration in ascending concentrations of ethanol and embedded in glycol methacrylate (GMA) resin. The specimens were sectioned at 5 to 10 µm and stained with TRAP and counter stained with methyl green for determination of cellular parameters. Bone histomorphometry was performed using an OsteoMeasure image analysis system connected via a video system to a suitable light microscope.

## Claims

1. Use of a statin for the manufacturing of pharmaceuticals for the inhibition of the formation of osteoclasts.

2. Use according to Claim 1, wherein the statin is selected from the group consisting of lovastatin, simvastatin, pravastatin, fluvastatin, atorvastatin, cerivastatin, mevastatin, rosuvastatin, and itavastatin.

3. Use according to Claim 2, wherein the statin is atorvastatin calcium.

4. A pharmaceutical composition comprising a therapeutically effective amount of a statin and a carrier.

5. The pharmaceutical composition according to Claim 4, wherein the statin is selected from the group consisting of lovastatin, simvastatin, pravastatin, fluvastatin, atorvastatin, cerivastatin, mevastatin, rosuvastatin, and itavastatin.

6. The pharmaceutical composition according to Claim 5, wherein the statin is atorvastatin calcium.

7. Use of a statin for the manufacturing of pharmaceuticals for treating or preventing a disease state by inhibiting the formation of osteoclasts.

8. The use according to Claim 7, wherein the disease state is selected from the group consisting of osteoporosis, Paget's disease, osteolysis, hypercalcemia of malignancy, osteogenesis imperfecta, osteoarthritis, alveolar bone loss, side effects of immunosuppressive therapy, and side effects of chronic glucocorticoid use.

9. Use according to Claim 8, wherein the disease state is osteoporosis.

10. Use according to Claim 9, wherein the statin is selected from the group consisting of. lovastatin, simvastatin, pravastatin, fluvastatin, atorvastatin, cerivastatin, mevastatin, rosuvastatin, and itavastatin.

11. Use according to Claim 10, wherein the statin is atorvastatin calcium.

12. A kit for achieving a therapeutic effect in a mammal comprising a therapeutically effective amount of a statin and a carrier in unit dosage form and a container for containing said dosage form.

13. The kit according to Claim 12, wherein the statin is atorvastatin.

14. The kit according to Claim 12, wherein the therapeutic effect is selected from the group consisting of. osteoporosis, Paget's disease, osteolysis, hypercalcemia of malignancy, osteogenesis imperfecta, osteoarthritis, alveolar bone loss, side effects of immunosuppressive therapy, and side effects of chronic glucocorticoid use.

15. The kit according to Claim 14, wherein the therapeutic effect is osteoporosis.
